# EUROPÄISCHE PATENTSCHRIFT

(11) **EP 3 455 349 B1**
(45) Veröffentlichungstag und Bekanntmachung des Hinweises auf die Patenterteilung: **24.06.2020**
(21) Anmeldenummer: 17754071.3
(22) Anmeldetag: 03.08.2017
(51) Int. Cl.: C12N 7/00, C07K 1/16

(54) **VERFAHREN ZUR AUFREINIGUNG VON VIREN ODER VIRUSÄHNLICHEN PARTIKELN UNTER VERWENDUNG EINER VERNETZTEN CELLULOSEHYDRAT-MEMBRAN**
METHOD FOR THE PURIFICATION OF VIRUSES OR VIRUS-LIKE PARTICLES USING A CROSSLINKED CELLULOSE HYDRATE MEMBRANE
PROCÉDÉ DE PURIFICATION DE VIRUS OU PSEUDO-PARTICULES VIRALES AU MOYEN D'UNE MEMBRANE EN HYDRATE DE CELLULOSE RÉTICULÉE

(30) Priorität: 01.09.2016 DE 102016010601
(43) Veröffentlichungstag der Anmeldung: 20.03.2019
(73) Patentinhaber: Sartorius Stedim Biotech GmbH, 37079 Göttingen (DE)
(72) Erfinder: PETERSEN, Rebecca, 67105 Schifferstadt (DE); SCHRÖDER-TITTMANN, Kathrin, 37075 Göttingen (DE); VILLAIN, Louis, 30451 Hannover (DE)
(74) Vertreter: Müller-Boré & Partner Patentanwälte PartG mbB
(86) Internationale Anmeldenummer: PCT/EP2017/000943
(87) Internationale Veröffentlichungsnummer: WO 2018/041389

(56) Entgegenhaltungen:
- WO-A1-2009/127285
- WO-A1-2012/169970
- WO-A1-2015/055269
- WO-A1-2017/076553

## Beschreibung

Die vorliegende Erfindung betrifft ein Verfahren zur Aufreinigung von Viren oder virusähnlichen Partikeln aus einer Lösung, wie zum Beispiel einer Bioprozesslösung, unter Verwendung einer vernetzten Cellulosehydrat-Membran sowie einen Kit zur Aufreinigung von Viren oder virusähnlichen Partikeln und dessen Verwendung.

Die Aufreinigung und Isolierung von Viren hat in den letzten Jahren, insbesondere in der pharmazeutischen Industrie, enorm an Bedeutung gewonnen. Nach der Kultivierung von Viren in Zellkulturen ist es notwendig, die Viren von den Kontaminanten, wie z.B. Wirtszellproteinen oder Desoxyribonukleinsäure (DNS), zu trennen, um sie in reiner Form weiterverwenden zu können. In biopharmazeutischen Produktionsprozessen treten DNS und andere Proteine der Wirte kultivierungsbedingt als Nebenprodukte auf. Diese zählen im Allgemeinen zu den Kontaminanten und müssen während der Aufarbeitung vom Endprodukt entfernt werden. Auch bei der Produktion von Viren werden DNS-Fragmente der Wirte oder Viren selbst freigesetzt, welche entfernt werden müssen.

Zur Aufreinigung von Viren oder von virusähnlichen Partikeln werden im Stand der Technik beispielsweise chromatographische Methoden eingesetzt. Sie beruhen im Allgemeinen auf der chemischen Funktionalität der Oberfläche der Festphase und der damit verbundenen Wechselwirkung zu den Biomolekülen in der mobilen Phase, die beim Durchströmen des Chromatographiemediums adsorptiv gebunden werden. Ein Nachteil dieser Verfahren ist, dass die Bindung der Viren durch die Affinität zu den Liganden erfolgt, was eine spezifische Eigenschaft der Viren darstellt. Für verschiedene Virusarten müssen dementsprechend verschiedene Chromatographiemedien mit spezifischen Liganden eingesetzt werden, um hohe Ausbeuten zu erzielen. Des Weiteren müssen bestimmte Umgebungsparameter, wie pH-Wert oder Leitfähigkeit, eingehalten werden.

Darüber hinaus sind im Stand der Technik Trennmethoden bekannt, die in erster Linie auf der Größe der Viren (genauer: des hydrodynamischen Volumens) basieren. Hierzu zählt die Dichtegradient-Ultrazentrifugation, welche beispielweise eine Cäsiumchlorid (CsCl)-Lösung verwendet, um u.a. Viren anhand ihrer Dichte zu trennen. Obwohl dieses Verfahren eine hochreine Auftrennung der unterschiedlichen Partikel liefert, weist dieses Verfahren verschiedene Nachteile auf. Insbesondere sind die Nachteile dieser Methode hohe Investitionskosten, lange Prozesszeiten, Kontamination durch CsCl, hohe Scherbeanspruchung und fehlende Skalierbarkeit.

Ein weiteres allgemein bekanntes Verfahren zur Aufreinigung von Viren aus Bioprozesslösungen ist die Größenausschlusschromatographie (size exclusion chromatography (SEC)) an Chromatographiegelen. Nachteile dieses Verfahrens sind eine niedrige Produktivität und Verringerung der Viruskonzentration (Verdünnung).

In WO 2012/169970 A1 wird ein Verfahren beschrieben, in dem biologische Produkte, wie Antikörper, Viren, Zellen, Zellorganellen oder Proteine, durch erzwungene Co-Hydratations-Chromatographie (constrained co-hydration chromatography) bzw. sterische Ausschlusschromatographie (steric exclusion chromatographie (SXC)) an Chromatographiematerial, insbesondere an Stärkepulver oder monolithischen Chromatographiematerialien, gereinigt werden.

Aufgrund der großen Volumina, die bei der Virusaufreinigung bewältigt werden müssen (ganz besonders für die Impfstoffherstellung), sind monolithische Chromatographiematerialien relativ teuer und weniger für den Einmalgebrauch (single-use) geeignet.

Der vorliegenden Erfindung liegt daher die Aufgabe zugrunde, eine kostengünstige, schnelle, robuste und selektive Methode zur Aufreinigung von Viren aus einer Lösung bereitzustellen, welche für eine große Palette an verschiedenen Arten von Viren oder virusähnlichen Partikeln anwendbar ist, unabhängig von ihren speziellen Oberflächeneigenschaften.

Diese Aufgabe wird durch die in den Ansprüchen gekennzeichneten Ausführungsformen der vorliegenden Erfindung gelöst.

Insbesondere stellt die vorliegende Erfindung ein Verfahren zur Aufreinigung von Viren oder virusähnlichen Partikeln aus einer Lösung, wie zum Beispiel einer Bioprozesslösung, bereit, welches die folgenden Schritte umfasst:
a) Bereitstellen einer Mischung M aus einer Lösung A, welche die aufzureinigenden Viren oder die virusähnlichen Partikel und Verunreinigungen enthält, und einer Lösung B, die Polyalkylenglykol mit zwei oder drei Kohlenstoffatomen in der Wiederholungseinheit enthält,
d) Beladen einer vernetzten Cellulosehydrat-Membran mit dieser Mischung M aus Schritt a), wobei sich die Viren oder die virusähnlichen Partikel an den äußeren und inneren Membranoberflächen anlagern und somit zurückgehalten werden, und
f) Eluieren der in der vernetzten Cellulosehydrat-Membran zurückgehaltenen Viren oder virusähnlichen Partikel mit einer Lösung, die kein Polyalkylenglykol mit zwei oder drei Kohlenstoffatomen in der Wiederholungseinheit enthält oder die mindestens 50% weniger Polyalkylenglykol mit zwei oder drei Kohlenstoffatomen in der Wiederholungseinheit enthält als die Mischung M aus Schritt a), so dass die Viren oder die virusähnlichen Partikel im Eluat in gereinigter Form erhalten werden.

Gemäß der vorliegenden Erfindung handelt es sich bei den Lösungen vorstehend und im Folgenden um wässrige Lösungen.

Überraschenderweise wurde festgestellt, dass durch Zusatz des Polyalkylenglykols mit zwei oder drei Kohlenstoffatomen in der Wiederholungseinheit zu der Lösung aus Viren oder virusähnlichen Partikeln und Beaufschlagung dieser Mischung auf die vernetzte Cellulosehydrat-Membran, die Viren oder virusähnlichen Partikel während des Durchströmens durch die Membran zurückgehalten werden und die Permeatlösung an Viren oder virusähnlichen Partikeln verarmt. Wie nachstehend genauer beschrieben wird, lässt sich durch Wahl der Molmasse und/oder der Konzentration des Polyalkylenglykols mit zwei oder drei Kohlenstoffatomen in der Wiederholungseinheit die Rückhaltung der Viren oder virusähnlichen Partikel in der Membran so optimieren, dass sich in der Permeatlösung nur noch Konzentrationen derselben von 1 % und kleiner nachweisen lassen.

Die beladene vernetzte Cellulosehydrat-Membran lässt sich in einer bevorzugten Ausführungsform durch Beaufschlagung mit einer Lösung, die so viel Polyalkylenglykol mit zwei oder drei Kohlenstoffatomen in der Wiederholungseinheit enthält, wie die Lösung, mit der die Membran vorher beladen wurde, in einem weiteren Schritt e) waschen, so dass die Verunreinigungen aus der Membran gespült werden. Die Viren bzw. die virusähnlichen Partikel verbleiben jedoch weiterhin in der Membran. Durch anschließende Beaufschlagung der Membran mit einer Lösung, die kein Polyalkylenglykol mit zwei oder drei Kohlenstoffatomen in der Wiederholungseinheit enthält oder die mindestens 50% weniger Polyalkylenglykol mit zwei oder drei Kohlenstoffatomen in der Wiederholungseinheit enthält als die Mischung M, werden die Viren oder die virusähnlichen Partikel wieder mobilisiert und in gereinigter Form eluiert.

Gemäß der vorliegenden Erfindung unterliegen die Viren keiner besonderen Einschränkung und schließen beispielsweise Influenzaviren, Gelbfieber-Viren, humane Papillomviren, Vacciniaviren, Adenoviren, Adeno-assoziierte Viren, Baculoviren, Hepatitisviren, Lentiviren, Polioviren, Rabiesviren, Rotaviren, Rubellaviren und Zikaviren ein. Unter virusähnlichen Partikeln, auch virus-like particles (VLP) genannt, werden erfindungsgemäß Viruspartikel verstanden, die keine Nukleinsäuren enthalten. Zu den virusähnlichen Partikeln zählen Viroide, Virusoide und Prionen. Nachfolgend werden die aufzureinigenden Viren und die virusähnlichen Partikel, welche in der Lösung, wie zum Beispiel in der Bioprozesslösung, enthalten sind, vereinfacht unter dem Begriff "Viren" zusammengefasst.

Mit dem erfindungsgemäßen Verfahren lassen sich die verschiedenen Arten von Viren unabhängig ihrer chemischen Affinitäten aufreinigen. Das Verfahren lässt sich mit wenigen Verfahrensparametern, wie Konzentration und/oder Molmasse des Polyalkylenglykols mit zwei oder drei Kohlenstoffatomen in der Wiederholungseinheit und/oder Kontaktzeit und/oder Verweilzeit für die jeweilige Virusart optimieren und kann damit als Plattformtechnologie eingesetzt werden.

Als Kontaktzeit wird das Zeitintervall definiert, in dem die Lösung A, welche die aufzureinigenden Viren oder die virusähnlichen Partikel und Verunreinigungen enthält, und die Lösung B, die Polyalkylenglykol mit zwei oder drei Kohlenstoffatomen in der Wiederholungseinheit enthält, zusammentreffen und Mischung M bilden, bis zu dem Zeitpunkt, an dem die Mischung M die Membran erreicht.

Als Verweilzeit wird das Zeitintervall definiert, in dem die Lösung M bei der jeweiligen absoluten Flussrate in der Membran verweilt. Damit entspricht die Verweilzeit dem Zeitintervall, welches den Viruspartikeln in der Lösung M für die Rückhaltung an der äußeren und inneren Oberfläche der Membran zur Verfügung steht.

Ein weiterer Vorteil des erfindungsgemäßen Verfahrens ist, dass Viren aus einer ungereinigten Lösung, vorzugsweise aber geklärten Bioprozesslösung, aufgereinigt werden können, ohne dass ein bestimmter pH-Wert oder eine bestimmte Leitfähigkeit eingestellt werden muss. So kann beispielsweise die Kultivierungslösung direkt nach der Zellabtrennung mit dem für die Viabilität des Virus optimalen Umgebungspuffer eingesetzt werden. Diese Pufferzusammensetzung kann während des ganzen Verfahrens beibehalten werden. Dies ist ein erheblicher Vorteil im Vergleich zu bekannten Methoden zur Virenaufreinigung, bei welchen oftmals hohe Salzkonzentrationen und spezielle Puffersysteme benötigt werden, welche einen negativen Einfluss auf die Viren haben können.

Zudem können mit dem erfindungsgemäßen Verfahren gegenüber herkömmlichen Chromatographieverfahren, wie zum Beispiel Affinitätschromatographie, sehr hohe relative Flussraten in Membranvolumen (MV) pro Minute von etwa 13 MV/min gegenüber 1 bis 4 MV/min bei Affinitätschromatographie-Säulen erreicht werden.

Überraschenderweise wurde zudem festgestellt, dass mit dem erfindungsgemäßen Verfahren unter Verwendung einer vernetzten Cellulosehydrat-Membran mit einer besonders bevorzugten Porengröße von 3 bis 5 µm eine deutlich höhere Leistung im Hinblick auf die Bindungskapazität gegenüber herkömmlichen hydrophilen Chromatographiematerialien sowie der Affinitätschromatographie erreicht wird.

Ein weiterer wichtiger Aspekt in der pharmazeutischen Industrie ist, dass sich diese vernetzte Cellulosehydrat-Membran vor dem Verfahren zudem in vorteilhafter Weise sanitisieren lässt, z.B. durch Laugen, insbesondere Natronlauge. Dementsprechend umfasst das vorliegende erfindungsgemäße Verfahren bevorzugt einen Schritt b) des Sanitisierens mit Lauge.

In einer bevorzugten Ausführungsform des erfindungsgemäßen Verfahrens werden die Lösung A, welche die aufzureinigenden Viren und Verunreinigungen enthält, und Lösung B, welche Polyalkylenglykol mit zwei oder drei Kohlenstoffatomen in der Wiederholungseinheit enthält, mit Hilfe eines Mischers, besonders bevorzugt eines statischen Mischers vermischt. Mit Hilfe des statischen Mischers ist in kurzer Zeit die Erzeugung einer homogenen Mischung aus den Lösungen A und B bei geringer Scherbeanspruchung der Viren möglich, weil keine beweglichen Mischerteile vorhanden sind.

Es ist weiterhin vorteilhaft, dass die Kontaktzeit der Viren oder der virusähnlichen Partikel mit der Lösung B, die Polyalkylenglykol mit zwei oder drei Kohlenstoffatomen in der Wiederholungseinheit enthält, vor dem Beladungsschritt weniger als eine Minute beträgt.

Das erfindungsgemäße Verfahren lässt sich für die jeweilige Größe und Form der zu reinigenden Viren durch Auswahl der Molmasse und/oder der Konzentration des Polyalkylenglykols mit zwei oder drei Kohlenstoffatomen in der Wiederholungseinheit anpassen. Generell gilt, je kleiner das Virus, desto größer ist die notwendige Konzentration an Polyalkylenglykol mit zwei oder drei Kohlenstoffatomen in der Wiederholungseinheit und/oder desto größer ist die notwendige Molmasse des Polyalkylenglykols mit zwei oder drei Kohlenstoffatomen in der Wiederholungseinheit.

Gemäß einer bevorzugten Ausführungsform beträgt die Konzentration an Polyalkylenglykol mit zwei oder drei Kohlenstoffatomen in der Wiederholungseinheit in der Mischung M, die mit der vernetzten Cellulosehydrat-Membran in Kontakt gebracht wird, 1 bis 20, vorzugsweise 5 bis 18, besonders bevorzugt 8 bis 15 Gew.-%, bezogen auf die Gesamtmasse der Mischung M.

Gemäß einer bevorzugten Ausführungsform ist das Polyalkylenglykol mit zwei oder drei Kohlenstoffatomen in der Wiederholungseinheit ausgewählt aus der Gruppe, bestehend aus Polyethylenglykol (PEG) und Polypropylenglykol oder Gemischen daraus.

Die mittlere Molmasse des Polyalkylenglykols mit zwei oder drei Kohlenstoffatomen in der Wiederholungseinheit wird in Abhängigkeit der aufzureinigenden Viren ausgewählt. Vorzugsweise beträgt die mittlere Molmasse des Polyalkylenglykols mit zwei oder drei Kohlenstoffatomen in der Wiederholungseinheit 600 g/mol bis 10.000 g/mol (Dalton). Besonders bevorzugt beträgt die mittlere Molmasse des Polyalkylenglykols mit zwei oder drei Kohlenstoffatomen in der Wiederholungseinheit mindestens 1.000 g/mol, mehr bevorzugt mindestens 2.000 g/mol, besonders bevorzugt mindestens 3.000 g/mol. Die Obergrenze der mittleren Molmasse des Polyalkylenglykols mit zwei oder drei Kohlenstoffatomen in der Wiederholungseinheit beträgt vorzugsweise 9.000 g/mol, mehr bevorzugt 8.000 g/mol.

Die Rückgewinnungsrate an aufgereinigten Viren im Eluat kann mehr als 50 % betragen, mehr bevorzugt 55 % oder mehr, und besonders bevorzugt 60 % oder mehr.

Weiterhin kann die Konzentration der Viren im Eluat um den Faktor ≥ 5, besonders bevorzugt ≥ 10 im Vergleich zur Ausgangslösung erhöht sein, wobei ein Bereich von Faktor 5 bis 10 auch bevorzugt ist.

Des Weiteren stellt die vorliegende Erfindung einen Kit zur Aufreinigung von Viren oder virusähnlichen Partikeln aus einer Lösung A, wie zum Beispiel einer Bioprozesslösung, die vorzugsweise geklärt ist, aber ungereinigt sein kann, bereit, mindestens umfassend eine Lösung B, die Polyalkylenglykol mit zwei oder drei Kohlenstoffatomen in der Wiederholungseinheit enthält, und eine vernetzte Cellulosehydrat-Membran. Dabei wird die Lösung A mit der Lösung B vermischt und als Mischung M durch die vernetzte Cellulosehydrat-Membran geführt. Die vorstehenden und nachfolgenden Ausführungen bezüglich des erfindungsgemäßen Verfahrens treffen daher auch auf das erfindungsgemäße Kit zu. Die vernetzte Cellulosehydrat-Membran ist vorzugsweise mehrlagig in einer Filtereinheit mit mindestens einem Einlauf für die Mischung M und einem Ablauf für Permeat angeordnet, wobei dem Einlauf ein Mischer, vorzugsweise ein statischer Mischer vorgeschaltet ist, der mit Vorratsbehältern für die Lösungen A und B verbunden ist. Für den Einmalgebrauch sind mindestens die vorstehend genannten Komponenten der Vorrichtung vorzugsweise aus Kunststoff gefertigt. Wiederverwendbare Teile von weiteren Komponenten, wie Pumpen, Ventile, Sensoren können aus anderen Materialien gefertigt sein.

Das erfindungsgemäße Kit wird vorzugsweise zur Aufreinigung von Viren oder virusähnlichen Partikeln in der Impfstoffherstellung als Einwegprodukt (Einmalprodukt) für eine einmalige Anwendung (single-use) verwendet.

Wie vorstehend beschrieben, betrifft die vorliegende Erfindung ein Verfahren zur Aufreinigung von Viren oder von virusähnlichen Partikeln auch aus einer Lösung, wie zum Beispiel einer Bioprozesslösung, insbesondere nach der Zellabtrennung (Klärung), in dem
a) eine Lösung A, welche Viren oder virusähnliche Partikel und Verunreinigungen enthält, mit einer Lösung B versetzt wird, welche Polyalkylenglykol mit zwei oder drei Kohlenstoffatomen in der Wiederholungseinheit enthält, und beide Lösungen vermischt werden,
d) diese so hergestellte Mischung M über eine vernetzte Cellulosehydrat-Membran geführt wird,
e) optional die Membran mit einer Lösung, welche so viel Polyalkylenglykol mit zwei oder drei Kohlenstoffatomen in der Wiederholungseinheit enthält wie die Mischung M, gewaschen wird und
f) anschließend die Membran mit einer Elutions-Lösung beaufschlagt wird, die kein Polyalkylenglykol mit zwei oder drei Kohlenstoffatomen in der Wiederholungseinheit enthält oder die mindestens 50% weniger Polyalkylenglykol mit zwei oder drei Kohlenstoffatomen in der Wiederholungseinheit enthält als die Mischung M, wodurch die Viren oder virusähnlichen Partikel in gereinigter Form eluiert werden.

In dem erfindungsgemäßen Verfahren werden Viren aus der Lösung, wie zum Beispiel der Bioprozesslösung abgetrennt. Erfindungsgemäß ist die (wässrige) Viruslösung nicht besonders beschränkt und kann neben den zu reinigenden Viren Verunreinigungen (Kontaminanten), wie beispielsweise Nukleinsäuren, Zellproteine, Endotoxine oder andere Kontaminanten enthalten. Für die Aufreinigung sind die chemischen Wechselwirkungen zwischen der Membranoberfläche und den zu reinigenden Viren vernachlässigbar.

Die im erfindungsgemäßen Verfahren verwendeten (wässrigen) Lösungen A und B können ferner einen auf die Viren abgestimmten Puffer enthalten. Wie vorstehend erwähnt, kann zudem die Salzkonzentration dieser Lösungen A und B beliebig in Abhängigkeit von der zu reinigenden Lösung eingestellt werden.

Erfindungsgemäß wird als Lösung A vorzugsweise die geklärte Bioprozesslösung eingesetzt und kann dementsprechend neben den Viren und Verunreinigungen (Kontaminanten) weitere Komponenten, wie beispielsweise einen Puffer und/oder Salze enthalten. Gleiches gilt für die Lösung B, die Polyalkylenglykol mit zwei oder drei Kohlenstoffatomen in der Wiederholungseinheit enthält. Dementsprechend könnten die Lösungen A und B auch als Pufferlösungen A und B bezeichnet werden.

Vorzugsweise umfasst das erfindungsgemäße Verfahren die folgenden Schritte:

### c) Äquilibrieren

In einem optionalen Äquilibrierungsschritt wird die vernetzte Cellulosehydrat-Membran mit einer pufferhaltigen Lösung B, welche Polyalkylenglykol mit zwei oder drei Kohlenstoffatomen in der Wiederholungseinheit enthält, gespült, bis sich eine konstante Leitfähigkeit und/oder ein konstantes UV-Signal im Permeat eingestellt haben. Die Pufferzusammensetzung und die Leitfähigkeit werden so ausgewählt, dass für die jeweilige Virusart optimale Bedingungen bestehen. Die gewählte Konzentration und Molmasse des Polyalkylenglykols mit zwei oder drei Kohlenstoffatomen in der Wiederholungseinheit ist abhängig von der Virusart.

### d) Beladen

Die Pufferlösung A (z.B. geklärte Zellkulturlösung) und die Pufferlösung B werden in einem entsprechenden Verhältnis vermischt (Schritt a)), so dass die gewünschte Konzentration an Polyalkylenglykol mit zwei oder drei Kohlenstoffatomen in der Wiederholungseinheit eingestellt wird. Anschließend wird diese Mischung M aus den Pufferlösungen A und B durch ein Membranmodul, welches die vernetzte Cellulosehydrat-Membran enthält, geführt und diese beladen.

### e) Waschen

In einem optionalen Schritt kann die Membran mit einer Lösung, die so viel Polyalkylenglykol mit zwei oder drei Kohlenstoffatomen in der Wiederholungseinheit enthält wie die Mischung M aus den Pufferlösungen A und B, gespült werden.

### f) Eluieren

Die Membran wird beaufschlagt mit einer Pufferlösung, die kein Polyalkylenglykol mit zwei öder drei Kohlenstoffatomen in der Wiederholungseinheit enthält oder die mindestens 50% weniger Polyalkylenglykol mit zwei oder drei Kohlenstoffatomen in der Wiederholungseinheit enthält als die Mischung M aus den Pufferlösungen A und B. Dabei eluieren die in der Membran zurückgehaltenen Viren im gereinigten und vorzugsweise aufkonzentrierten Zustand als Eluat aus der Membran.

Es ist bekannt, dass mit steigender Konzentration an Polyalkylenglykol mit zwei oder drei Kohlenstoffatomen in der Wiederholungseinheit die Viskosität der Lösung zunimmt. Beispielsweise hat eine 10 %-ige Lösung aus Polyalkylenglykol mit zwei oder drei Kohlenstoffatomen in der Wiederholungseinheit mit einer Molmasse von 6.000 g/mol wie zum Beispiel das Polyethylenglykol (PEG) PEG-6.000 in Wasser bei 25°C bereits eine mehr als vier Mal höhere Viskosität als Wasser, wohingegen die Viskosität einer 20 %-igen Lösung aus Polyethylenglykol PEG-6.000 in Wasser mehr als elf Mal höher ist als Wasser. Eine höhere Viskosität geht jedoch mit einer Abnahme der Durchflussrate einher und wirkt sich dementsprechend negativ auf die Produktivität des Trennungsprozesses aus. Andererseits ermöglicht die Gegenwart von Polyalkylenglykol mit zwei oder drei Kohlenstoffatomen in der Wiederholungseinheit in der Lösung B, dass Viren von der Membran trotz der relativ großen Porengröße zurückgehalten werden.

Es hat sich gezeigt, dass für sehr kleine Viren, wie beispielsweise Adeno-assoziiertem Virus (AAV2), oder für kleine Phagen, wie beispielsweise phi X 174, welche einen durchschnittlichen Partikeldurchmesser von ca. 22 nm bzw. 25 nm aufweisen, eine Konzentration an Polyalkylenglykol mit zwei oder drei Kohlenstoffatomen in der Wiederholungseinheit wie zum Beispiel PEG von 10 % oder mehr, vorzugsweise von 11 % oder mehr, besonders bevorzugt von 12 % oder mehr, bei einer mittleren Molmasse von 6.000 g/mol, notwendig ist, um hohe Ausbeuten und Reinheitsgrade zu erzielen. Bei diesen Konzentrationen liegen jedoch Bedingungen vor, bei denen die Viren dieser Größe normalerweise in Anwesenheit von Polyalkylenglykol mit zwei oder drei Kohlenstoffatomen in der Wiederholungseinheit, wie zum Beispiel PEG ausgefällt werden. Es wurde aber überraschenderweise gefunden, dass durch den Einsatz eines Mischers und bevorzugt eines statischen Mischers und der Realisierung sehr kurzer Kontaktzeiten vor Beaufschlagung auf die Membran ein Ausfällen der Viren vermieden wird und hohe Rückgewinnungsraten und Reinheitsgrade erzielt werden können.

Gemäß der vorliegenden Erfindung beträgt die Kontaktzeit der Viren mit der Lösung B, welche Polyalkylenglykol mit zwei oder drei Kohlenstoffatomen in der Wiederholungseinheit enthält, vor dem Beladungsschritt vorzugsweise weniger als eine Minute. Dies wird vorzugsweise dadurch erreicht, dass die Lösungen A und B mit Hilfe eines Mischers und besonders bevorzugt eines statischen Mischers intensiv vermischt werden und direkt nach dem Mischer durch das Membranmodul mit der vernetzten Cellulosehydrat-Membran geführt werden. Dabei ist es vorteilhafter Weise möglich, dass ein Ausfällen der Viren vermieden werden kann.

Ein weiterer wichtiger Aspekt der vorliegenden Erfindung liegt darin, dass die Aufreinigung der Viren anhand des erfindungsgemäßen Verfahrens erfolgen kann, indem beispielsweise eine Kultivierungslösung direkt nach der Zellabtrennung eingesetzt werden kann, ohne dass vorher ein bestimmter pH-Wert oder eine bestimmte Leitfähigkeit eingestellt werden muss. Dementsprechend kann eine für die Viabilität des Virus optimale Pufferzusammensetzung während des ganzen Verfahrens verwendet werden.

Aus WO 2012/169970 A1 ist bekannt, dass eine hohe Salzkonzentration bei der SXC an Stärkepulver oder monolithischen Chromatographiematerialien zu einem dramatischen Rückgang der Bindungsfähigkeit führt. Gemäß der vorliegenden Erfindung hat sich gezeigt, dass selbst bei hohen Salzkonzentrationen, beispielsweise bei einer 500 mM NaCl Lösung, keine signifikanten Einbußen in den Wiederfindungsraten eintreten.

Gemäß der vorliegenden Erfindung erfolgt die Aufreinigung der Viren mit Hilfe einer vernetzten Cellulosehydrat-Membran. Erfindungsgemäß werden poröse, flächig ausgebildete Cellulosehydrat-Membranen verwendet, welche sich somit deutlich von chromatographischen Säulen etc. unterscheiden. Diese sind nicht besonders beschränkt, solange es sich um vernetzte Cellulosehydrat-Membranen mit vorzugsweise einer Porengröße von 0,5 bis 10 µm, besonders bevorzugt von 3 bis 5 µm, handelt. Solche Membranen sind im Stand der Technik bekannt und auch kommerziell erhältlich, beispielsweise als Hydrosart® Membran von der Patentanmelderin.

Die vernetzte Cellulosehydrat-Membran wird nach WO 1995/032793 A1 unter Verwendung mindestens eines Vernetzungsmittels, welches bzw. welche mindestens 2 funktionelle Gruppen im Molekül aufweist bzw. aufweisen, die mit den Hydroxylgruppen der Cellulose reaktiv sind, hergestellt. Solche Vernetzungsmittel können Diepoxide, wie 5,6-Diepoxy-hexan, Glycidylether, wie 1,4-Butandioldiglycidylether, Ethylenglycoldiglycidylether, Glycerindiglycidylether, Polyethylenglycoldiglycidylether, Epichlorhydrin, Epibromhydrin, Alkylen-Dihalogen- oder Hydroxyalkylen-Dihalogen-Verbindungen sein.

Die Verwendung einer vernetzten Cellulosehydrat-Membran mit einer Porengröße von vorzugsweise 0,5 bis 10 µm, besonders bevorzugt 3 bis 5 µm erlaubt zudem eine hohe Beladung der Membran, was zu einer hohen Bindungskapazität gegenüber herkömmlichen hydrophilen Chromatographiematerialien führt. Wie in den nachstehenden Beispielen belegt, ist die Bindungskapazität bezogen auf das Volumen der Festphase gemäß einer erfindungsgemäßen Ausführungsform mindestens um den Faktor 10 größer als bei herkömmlichen Chromatographiematerialien, wie Affinitätschromatographiesäulen.

Die Verwendung einer vernetzten Cellulosehydrat-Membran mit einer Porengröße größer 1 µm ermöglicht deutlich höhere Permeabilitäten für die viskosen Lösungen, welche Polyalkylenglykol mit zwei oder drei Kohlenstoffatomen in der Wiederholungseinheit enthalten.

Im Vergleich zu unvernetzten Cellulosehydrat-Membranen zeichnen sich die vernetzten Cellulosehydrat-Membranen durch deutlich höhere Permeabilitäten nach der Sanitisierung aus.

Die vernetzte Cellulosehydrat-Membran kann erfindungsgemäß sowohl als verstärkte als auch unverstärkte Membran verwendet werden, wobei die Verwendung verstärkter Cellulosehydrat-Membranen bevorzugt ist. Geeignete Verstärkungen sind beispielsweise Vliese oder Gewebe.

Wie vorstehend beschrieben, können mit dem erfindungsgemäßen Verfahren im Vergleich zu herkömmlichen Chromatographieverfahren sehr hohe absolute Flussraten erreicht werden, wodurch eine Verringerung der Kontaktzeiten vor Beaufschlagung auf die Membran erzielt werden kann. Dadurch kann ein Ausfällen der Viren vermieden und hohe Rückgewinnungsraten und Reinheitsgrade erzielt werden.

Gemäß einer bevorzugten Ausführungsform der vorliegenden Erfindung beträgt die Rückgewinnungsrate an aufgereinigten Viren im Eluat mehr als 50 %. Im Vergleich zu einem herkömmlichen Affinitätschromatographie-Verfahren wird mit dem erfindungsgemäßen Verfahren eine Konzentration der Viren im Elutat erzielt, die um beispielsweise Faktor 10 höher ist, als in der Ausgangslösung.

Die vorliegende Erfindung wird anhand der folgenden, nicht einschränkenden Beispiele und Figuren näher erläutert.
Figur 1 zeigt beispielhaft den Verlauf der Aufreinigung von phi X 174 aus dem Ausführungsbeispiel 1 mit den Schritten Äquilibrieren, Beladen, Waschen und Eluieren.
Figur 2 zeigt die Reinigungseffizienz des erfindungsgemäßen Verfahrens beispielhaft an phi X 174, gemessen mit der Größenausschlusschromatographie anhand des Chromatogramms vor und nach der Aufreinigung.
Figur 3 zeigt die dynamische Bindungskapazität bei der Reinigung von Bakteriophagen phi X 174 aus dem Ausführungsbeispiel 3.

### Beispiele

### Ausführungsbeispiel 1: Reinigung von Bakteriophagen phi X 174 aus der geklärten Prozesslösung

Die zu reinigenden Phagen wurden zunächst durch Infektion einer Bakterienkultur (E.coli C600) in TSB Puffer erzeugt. Nach Abtrennung der Bakterien mittels Filtration durch zwei Filtersysteme (SartopurePP2 & Sartopore 2, beide von Sartorius Stedim Biotech GmbH) lag eine Phagen-Konzentration von 3,8 x 10¹¹ Partikel/mL vor. Diese Lösung wird nachfolgend als "Phagenlösung" bezeichnet.

Für die chromatographische Reinigung wurde eine Hydrosart® Membran (Sartorius Stedim Biotech GmbH), d.h. eine vernetzte Cellulosehydrat-Membran, mit einer Porengröße von 3 bis 5 µm verwendet. Eine die Cellulosehydrat-Membran enthaltene Filtrationseinheit Vivapure® System (SSB, Göttingen) mit einem Membranvolumen (MV) von 0,023 mL wurde an ein Äkta-System (Äkta Prime, GE Healthcare Life Science) angeschlossen. Die absolute Flussrate lag über den Versuch konstant bei 0,3 mL/min, das entspricht einer relativen Flussrate von ca. 10 MV/min. Zunächst wurde die Membran mit einem Gemisch aus Puffer A (50 mM Tris (Merck Millipore), pH 7,0, 150 mM NaCl (Merck Millipore)) und Puffer B (50 mM Tris, pH 7,0, 150 mM NaCl, 16 Gew.-% PEG-6.000 (Mw=6.000 g/mol, Carl Roth)) im Verhältnis 1:4 äquilibriert. Die finale Konzentration an PEG-6.000 in der Mischung betrug 12 Gew.-%.

Für die Beladung wurde Puffer A durch die Phagenlösung ersetzt. Der Mischvorgang erfolgte in-line in einem statischen Mischer des Äkta-Systems. Nach Beladung von insgesamt 55 mL Lösung (13,7 mL Phagenlösung und 41,3 mL Puffer B) wurde die Membran gewaschen. Hierfür wurde wieder ein Gemisch aus Puffer A und Puffer B im Verhältnis 1:4 verwendet. Die Elution der Phagenpartikel von der Membran erfolgte durch Verwendung von 100 % Puffer A. Die Wiederfindung an Phagen lag bei 81,6 % (± 3 %).

Eine Quantifizierung der Viruspartikel erfolgte mittels quantitativer Echtzeit-PCR (real-time PCR) an einem Gerät von Agilent Technologies (Mx3005P qPCR System) unter Verwendung des Mastermixes Brilliant III Ultra-Fast SYBR® Green QPCR von Agilent Technologies. Als Referenz wurde ein Plasmid verwendet, welches die Sequenz des phi X 174 Phagen trägt (Fermentas GmbH).

Um die Abreicherung von Kontaminanten bei gleichzeitiger Anreicherung von Phagenpartikeln zu quantifizieren, wurde Größenausschlusschromatographie (SEC) durchgeführt. Hierfür wurde eine SEC-Säule (Yarra 3000, Phenomenex) an ein HPLC System (UltiMate 3000, Thermo Scientific Dionex) angeschlossen und jeweils 100 µl Probe aufgetragen. Die absolute Flussrate betrug 1 ml/min und der Laufpuffer setzte sich wie folgt zusammen: 100 mM Natriumphosphat und 100 mM Natriumsulfat (pH = 6,6).

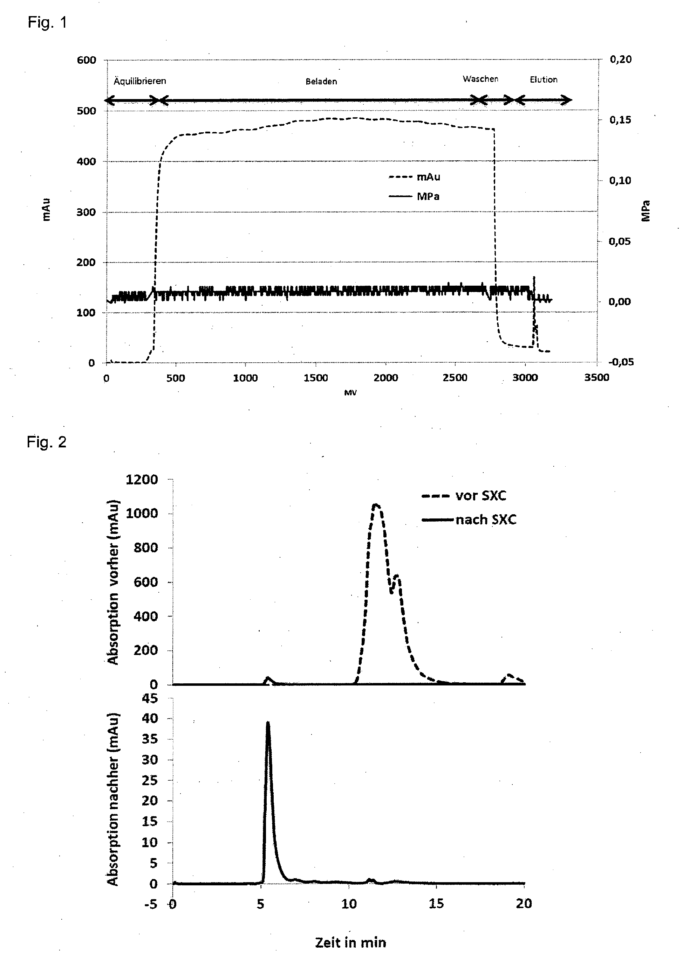

Fig. 1 zeigt beispielhaft den Verlauf der Aufreinigung von phi X 174 aus dem Ausführungsbeispiel 1 mit den Schritten Äquilibrieren, Beladen, Waschen und Eluieren.

Fig. 2 zeigt die Reinigungseffizienz des Verfahrens beispielhaft an phi X 174, gemessen mit der Größenausschlusschromatographie anhand des Chromatogramms vor und nach der Aufreinigung. Die Verunreinigungen (großer Peak bei 12 min) verschwinden vollständig und das Chromatogramm zeigt nur noch den reinen Phagen-Peak bei ca. 6 min.

### Ausführungsbeispiel 2: Reinigung von Bakteriophagen phi X 174 aus der geklärten Prozesslösung mit kürzerer Kontaktzeit

Die Methode wurde entsprechend dem Ausführungsbeispiel 1 an zwei weiteren vernetzte Cellulosehydrat-Membran enthaltenden Filtrationseinheiten durchgeführt, welche gegenüber dem Ausführungsbeispiel 1 ein höheres Membranvolumen von a) 0,046 mL und b) 0,56 mL besaßen. Die relative Flussrate wurde wie bei Ausführungsbeispiel 1 jeweils auf ca. 10 MV/min eingestellt. Aufgrund der veränderten Membranvolumina gegenüber dem Ausführungsbeispiel 1 veränderten sich die jeweiligen absoluten Flussraten von 0,3 mL/min in Ausführungsbeispiel 1 auf a) 0,6 mL/min und b) 5,6 mL/min. Gleichzeitig stellte sich mit der Erhöhung der absoluten Flussrate die gewünschte Verringerung der Kontaktzeit von 16,9 min im Ausführungsbeispiel 1 auf a) 8,3 min und b) 0,93 min ein. Die Versuche wurden jeweils zweimal durchgeführt, die Ergebnisse werden in Tabelle 1 dargestellt.

Eine Verringerung der Kontaktzeit von 16,9 min in Ausführungsbeispiel 1 auf 2a) 8,3 min zeigte keinen signifikanten Effekt auf die Rückgewinnungsrate und Bindungskapazität. Jedoch führte die Verringerung der Kontaktzeit auf weniger als 1 min in 2b) zu einer deutlichen Verbesserung der Rückgewinnungsrate auf mehr als 97 % bei gleichzeitig ca. 0 % Wiederfindungsrate im Permeat.

**Tabelle 1:**

| Übersicht über Parameter und Ergebnisse bei der Aufreinigung von Bakteriophagen phi X 174 an vernetzten Cellulosehydrat-Membranen für verschiedene Membranvolumina (MV) | | | |
|---|---|---|---|
| | **Beispiel 1** | **Beispiel 2a)** | **Beispiel 2b)** |
| MV in mL | 0,023 | 0,046 | 0,56 |
| absolute Flussrate in mL/min | 0,3 | 0,6 | 5,6 |
| Kontaktzeit in min | 16,9 | 8,4 | 0,93 |
| Wiederfindung im Eluat in % | 81,6 ± 3 | 79,0 ± 0 | 97,4 ± 0,7 |
| Wiederfindung im Permeat in % | 0,7 ± 0,3 | 1,0 ± 0 | 0,05 ± 0,01 |
| Bindungskapazität (Partikel/mL MV) | 1,6 x 10¹⁴ | 1,7 x 10¹⁴ | 2,5 x 10¹⁴ |

### Ausführungsbeispiel 3: Reinigung von Bakteriophagen phi X 174 aus der geklärten Prozesslösung für verschiedene Verweilzeiten in der Membran

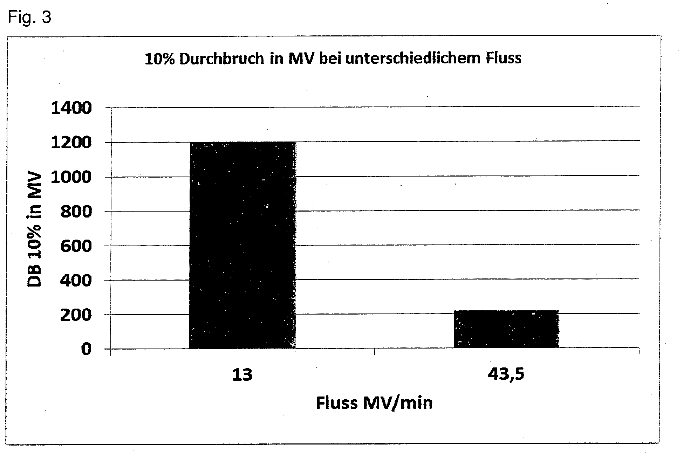

Die Methode wurde entsprechend dem Ausführungsbeispiel 1 an einer die vernetzte Cellulosehydrat-Membran enthaltenden Filtrationseinheit Vivapure® System (SSB, Göttingen) mit einem Membranvolumen von 0,023 mL durchgeführt, jedoch mit einer absoluten Flussrate von 1 ml/min, wobei sich die relative Flussrate gegenüber dem Ausführungsbeispiel 1 von 13 MV/min auf 43,5 MV/min erhöhte, dadurch verringerte sich gleichzeitig die Verweilzeit von 4,6 sec auf 1,4 sec. Berechnet wurde die dynamische Bindungskapazität (DBK 10%), bei der ein Durchbruch der Phagenpartikel von 10%, bezogen auf die Ausgangslösung zu detektieren war. Die Ergebnisse sind in Tabelle 2 und in Fig. 3 dargestellt. Bei einer Verweilzeit von 4,6 sec erfolgte der Durchbruch der Phagenpartikel von 10%, bezogen auf die Ausgangslösung bei etwa 1200 Membranvolumen (MV), bei einer Verweilzeit von nur 1,4 sec schon bei ca. 200 Membranvolumen (MV). Durch eine Vergrößerung der Verweilzeit konnte also das maximale Volumen der Phagenlösung, welches sich über die Filtrationseinheit aufreinigen lässt, bevor der Durchbruch der Phagenpartikel von 10% bezogen auf die Ausgangslösung erreicht wird, um den Faktor 6 erhöht werden.

**Tabelle 2:**

| Übersicht über Parameter und Ergebnisse bei der Aufreinigung von Bakteriophagen phi X 174 an vernetzten Cellulosehydrat-Membranen für verschiedene Verweilzeiten | | |
|---|---|---|
| | Beispiel 1 | Beispiel 3 |
| MV in mL | 0,023 | 0,023 |
| absolute Flussrate in mL/min | 0,3 | 1,0 |
| Relative Flussrate in MV/min | 13 | 43,5 |
| Verweilzeit in sec | 4,6 | 1,4 |
| DBK 10% in MV | 1200 | 200 |

### Ausführungsbeispiel 4: Reinigung von Bakteriophagen phi X 174 aus der geklärten Prozesslösung für verschiedene Leitfähigkeiten

Die Methode wurde entsprechend dem Ausführungsbeispiel 1 an einer die vernetzte Cellulosehydrat-Membran enthaltenden Filtrationseinheit Vivapure® System (SSB, Göttingen) mit einem Membranvolumen von 0,023 mL durchgeführt, jedoch mit einer Salzkonzentration in Puffer A und B von 500 mM Natriumchlorid. Die im Rahmen des Versuchsplans eingesetzte PEG-Konzentration betrug bei dieser Versuchsreihe 11 %, daher sind die Rückgewinnungsraten insgesamt niedriger als bei 12 % PEG. Der Versuch wurde zweimal durchgeführt. Ein signifikanter Einfluss der Salzkonzentration auf die Wiederfindungsrate liegt nicht vor, wie dies anhand der in Tabelle 3 gezeigten Ergebnisse zu sehen ist.

**Tabelle 3:**

| Rückgewinnungsrate bei der Reinigung von Bakteriophagen phi X 174 aus der geklärten Prozesslösung für verschiedene Leitfähigkeiten | | |
|---|---|---|
| | Beispiel 1 | Beispiel 4 |
| MV in mL | 0,023 | 0,023 |
| absolute Flussrate in mL/min | 0,3 | 0,3 |
| Konzentration NaCl in mMol | 150 | 500 |
| Rückgewinnung im Eluat in % | 64,6 ± 1,0 | 72,6 ± 2,8 |

### Ausführungsbeispiel 5: Reinigung von Adeno-assoziiertem Virus (AAV2) aus biotechnologischer Lösung

Die Adeno-assoziierten Viruspartikel (AAV2) wurden unter Verwendung von HEK 293 T (ATCC® CRL-3216™) hergestellt. Die Zellen wurden unter Verwendung von Plasmiden transfiziert. Die AAV2-Stammlösung wurde mit Benzonase behandelt (30 Einheiten/ml), durch Zentrifugation (300 x g, mit g = Erdbeschleunigung, für 20 min bei 4°C) geklärt und durch 0,2 µm Sterilfilter (Minisart®, Sartorius Stedim Biotech GmbH) gefiltert. Diese Lösung wird "AAV2-Lösung" genannt.

Die Reinigung erfolgte an einer die vernetzte Cellulosehydrat-Membran enthaltenden Filtrationseinheit Vivapure® (SSB, Göttingen) mit einer Porengröße von 3 bis 5 µm und einem Membranvolumen von 0,046 mL, angeschlossen an ein Äkta-System (Äkta Prime, GE Healthcare Life Science). Die absolute Flussrate lag über den Versuch konstant bei 0,6 mL/min. Zunächst wurde die Membran mit einem Gemisch aus Puffer A (0,5 M NaCl, 0,05 M Tris, pH 7,0, Leitfähigkeit 53,7 mS/cm) und Puffer B (16 Gew.-% PEG-6.000, 0,5 M NaCl, 0,05 M Tris, pH = 7,0, Leitfähigkeit 24,4 mS/cm) im Verhältnis 1:4 äquilibriert. Die finale Konzentration an PEG-6000 in der Mischung betrug 12 Gew.-%.

Für die Bindung der AAV2-Partikel wurde Puffer A durch die AAV2-Lösung ersetzt. Der Mischvorgang erfolgte in-line in einem statischen Mischer des Äkta-Systems. Nach Beladung von insgesamt 55 mL Lösung (13,7 mL AAV2-Lösung und 41,3 mL Puffer B) wurde die Membran gewaschen. Hierfür wurde wieder ein Gemisch aus Puffer A und Puffer B im Verhältnis 1:4 verwendet. Die Elution der AAV2-Partikel von der Membran erfolgte durch Verwendung von 100 % Puffer A.

Eine Quantifizierung der AAV2-Partikel erfolgte mittels quantitativer Echtzeit-PCR (real-time PCR) an einem Gerät von LightCycler® 480 (Roche Life Science) unter Verwendung des Mastermixes LightCycler Faststart Master SYBR Green. Als Referenz wurde ein intern hergestelltes Plasmid verwendet, welches die Sequenz des AAV2-Partikels trägt.

Die Rückgewinnungs- bzw. Wiederfindungsrate des aufgereinigten AAV2-Partikel betrug 81,8 %, bei gleichzeitigem Durchbruch von 0,18 % im Permeat und 0,15 % in der Waschlösung. Die Bindungskapazität betrug 2,52 x 10¹³ Partikel je mL Membranvolumen.

Im Vergleich dazu wurde die Aufreinigung der AAV2-Partikel aus derselben biotechnologischen Lösung mit der Methode der Affinitätsschromatographie durchgeführt. Dazu wurde die Viruslösung über eine Chromatographiesäule, gepackt mit AVB Sepharose Affinity Resin, mit einem Säulenvolumen von 0,2 ml bei einer Flussrate von 1,2 ml/min geführt.

Die Rückgewinnungs- bzw. Wiederfindungsrate des durch Affinitätschromatographie aufgereinigten AAV2-Partikel betrug 30,0 %. Die Bindungskapazität betrug 1,82 x 10¹² Partikel je mL Membranvolumen.

Die Ergebnisse in Tabelle 4 zeigen, dass bei der erfindungsgemäß beschriebenen Aufreinigungsmethode eine um Faktor 10 höhere Bindungskapazität gegenüber herkömmlicher Affinitätschromatographie erzielt wird. Ebenso wird eine höhere Rückgewinnungsrate (recovery) erreicht.

**Tabelle 4:**

| Übersicht über Parameter und Ergebnisse bei der Aufreinigung von AAV2 an vernetzten Cellulosehydrat-Membranen an Hydrosart® gegenüber der herkömmlichen Methode durch Affinitätschromatographie | | |
|---|---|---|
| | **Aufreinigung nach erfindungsgemäßen Verfahren** | **Affinitätschromatographie** |
| MV in mL | 0,046 | 0,2 |
| Partikelkonzentration in der Ausgangslösung in Partikel/mL | 1,42 x 10¹² | 1,42 x 10¹² |
| Partikelkonzentration im Durchbruch in Partikel/mL | 2,62 x 10⁹ | 2,08 x 10¹¹ |
| Partikelkonzentration im Eluat in Partikel/mL | 1,16 x 10¹² | 3,64 x 10¹¹ |
| Bindungskapazität in Partikel/mL MV | 2,52 x 10¹³ | 1,82 x 10¹² |
| Wiederfindungsrate im Eluat in % | 81,8 | 30,0 |

### Ausführungsbeispiel 6: Reinigung von Adeno Virus Typ5 aus biotechnologischer Lösung

Die Adeno Viruspartikel Typ 5 (AD5) wurden unter Verwendung von HEK 293 (ATCC® CRL-1573™) hergestellt. Die AD5-Stammlösung wurde mit Benzonase behandelt (50 Einheiten/ml), durch Zentrifugation (300 g für 20 min bei 4°C) geklärt und durch 0,45 µm Sterilfilter (Minisart®, Sartorius Stedim Biotech GmbH) gefiltert, wodurch eine Konzentration von 4,5 x 10⁹ Partikel/mL erhalten wurde. Diese Lösung wird "AD5-Lösung" genannt.

Die Reinigung erfolgte an einer die vernetzte Cellulosehydrat-Membran enthaltenden Filtrationseinheit Vivapure® (SSB, Göttingen) mit einer Porengröße von 3 bis 5 µm und einem Membranvolumen von 0,023 mL, angeschlossen an ein Äkta-System. Die absolute Flussrate lag über den Versuch konstant bei 0,3 mL/min. Zunächst wurde die Membran mit einem Gemisch aus Puffer A (0,5 M NaCl, 0,05 M Tris, pH = 7,0, Leitfähigkeit 53,2 mS/cm) und Puffer B (13,3 Gew.-% PEG-6000, 0,5 M NaCl, 0,05 M Tris, pH = 7,0, Leitfähigkeit 28,4 mS/cm) im Verhältnis 1:4 äquilibriert. Die finale Konzentration an PEG-6.000 in der Mischung betrug 10 Gew.-%. Für die Bindung der AD5-Partikel wurde Puffer A durch die AD5-Lösung ersetzt. Der Mischvorgang erfolgte in-line in einem statischen Mischer des Äkta-Systems. Nach Beladung von insgesamt 55 mL Lösung (13,7 mL AD5-Lösung und 41,3 mL Puffer B) wurde die Membran gewaschen. Hierfür wurde wieder ein Gemisch aus Puffer A und Puffer B im Verhältnis 1:4 verwendet. Die Elution der AD5-Partikel von der Membran erfolgte durch Verwendung von 100 % Puffer A.

Eine Quantifizierung der AD5-Partikel erfolgte mittels quantitativer Echtzeit-PCR (real-time PCR) an einem Gerät von Roche Life Science LC480 unter Verwendung des Mastermixes Brilliant III Ultra-Fast SYBR® Green QPCR. Als Referenz wurde ein interner Standard AD5 viraler DNA verwendet.

Die Rückgewinnungsrate des aufgereinigten AD5-Virus aus der Elution betrug 82,3 % bei einer Bindungskapazität von 1,4 x 10¹² Partikel/ml Membranvolumen.

### Ausführungsbeispiel 7: Einfluss der Porengröße einer vernetzten Cellulosehydrat-Membran auf den Leistungsparameter Permeabilität

Vernetzte Cellulosehydrat-Membranen vom Typ Hydrosart® Membran (Sartorius Stedim Biotech GmbH) mit verschiedenen Porengrößen wurden hinsichtlich ihrer Permeabilitäten bei der Beaufschlagung PEG-haltiger Lösungen, wie sie im erfindungsgemäßen Verfahren vorliegen, verglichen. Dazu wurden Membranen mit zwei verschiedenen Porengrößen unter Beaufschlagung von 1 bar Druck mit der PEG-haltigen Lösung durchströmt, der Durchlauf wurde aufgefangen und volumetrisch bestimmt. Die verwendete Lösung entsprach der Mischung M aus Puffer A und B gemäß Ausführungsbeispiel 1 und enthielt 12 % PEG.

Zum Vergleich wurde die Permeabilität der Membran mit der Porengröße 3 bis 5 µm auf 100% festgelegt. Bei der Membran mit einer Porengröße von 1 µm lag die Permeabilität bei nur 30,8% verglichen mit der Permeabilität der Membran mit einer Porengröße von 3 bis 5 µm.

**Tabelle 5:**

| Übersicht über Parameter und Messwerte bei der Bestimmung des Einflusses der Porengröße auf den Leistungsparameter Permeabilität für venetzte Cellulosehydrat-Membranen bei der Durchströmung mit der PEG-haltigen Mischung M aus dem Ausführungsbeispiel 1 | | |
|---|---|---|
| | Membran 1 | Membran 2 |
| Porengröße in µm | 3 bis 5 | 1 |
| Mischung M mit 12% PEG Permeabilität (ml/min*bar*cm²) | 86,6 | 35,7 |
| Vergleich % | 100 | 30,8 |

### Ausführungsbeispiel 8: Einfluss der Sanitisierung auf den Leistungsparameter Permeabilität im Vergleich von vernetzter und unvernetzter Cellulosehydrat-Membran

Eine vernetzte Cellulosehydrat-Membran vom Typ Hydrosart® Membran (Sartorius Stedim Biotech GmbH) mit der Porengröße 1 µm wurde hinsichtlich ihrer Permeabilität bei der Beaufschlagung mit PEG-haltigen Lösungen nach der Sanitisierung mit einer unvernetzten Cellulosehydrat-Membran mit der Porengröße 1 µm verglichen.

Dazu wurden die Membranen durch Beaufschlagung mit 1 N Natronlauge für 30 min sanitisiert und mit Puffer A (ca. 150 MV) äquilibriert. Anschließend wurden Permeabilitäten der PEG-haltigen Mischung M bestimmt, wie es in Ausführungsbeispiel 7 beschrieben ist.

**Tabelle 6:**

| Übersicht über Parameter und Messwerte bei der Bestimmung des Leistungsparameters Permeabilität für vernetzte und unvernetzte Cellulosehydrat-Membranen nach der Sanitisierung mit Natronlauge | | |
|---|---|---|
| | Membran 2 | Membran 3 |
| | vernetzt | unvernetzt |
| Mischung M mit 12% PEG Permeabilität (ml/min*bar*cm²) | 35,5 | 5,56 |
| Vergleich % | 100 | 14,1 |

Nach Sanitisierung ist die Permeabilität der unvernetzten Cellulosehydrat-Membran für die PEG-haltige Mischung M gemäß Ausführungsbeispiel 1 nur noch 14% im Vergleich zur vernetzten Cellulosehydrat-Membran.

## Patentansprüche

1. Verfahren zur Aufreinigung von Viren oder virusähnlichen Partikeln aus einer Lösung, umfassend die folgenden Schritte:
a) Bereitstellen einer Mischung M aus einer Lösung A, welche die aufzureinigenden Viren oder die virusähnlichen Partikel und Verunreinigungen enthält, und einer Lösung B, die ein Polyalkylenglykol mit zwei oder drei Kohlenstoffatomen in der Wiederholungseinheit enthält,
d) Beladen einer vernetzten Cellulosehydrat-Membran mit der Mischung M aus Schritt a), wobei sich die Viren oder die virusähnlichen Partikel an den äußeren und inneren Membranoberflächen anlagern und zurückgehalten werden,
und
f) Eluieren der in der vernetzten Cellulosehydrat-Membran zurückgehaltenen Viren oder virusähnlichen Partikel mit einer Lösung, die kein Polyalkylenglykol mit zwei oder drei Kohlenstoffatomen in der Wiederholungseinheit enthält oder die mindestens 50% weniger Polyalkylenglykol mit zwei oder drei Kohlenstoffatomen in der Wiederholungseinheit enthält als die Mischung M aus Schritt a), so dass die Viren oder die virusähnlichen Partikel im Eluat in gereinigter Form erhalten werden.

2. Verfahren nach Anspruch 1, wobei als Lösung A eine ungereinigte, geklärte Bioprozesslösung eingesetzt wird.

3. Verfahren nach Anspruch 1 oder 2, wobei dem Schritt f) ein weiterer Schritt e) vorangestellt wird, in dem die beladene vernetzte Cellulosehydrat-Membran mit einer Lösung gewaschen wird, die so viel Polyalkylenglykol mit zwei oder drei Kohlenstoffatomen in der Wiederholungseinheit wie die Mischung M aus Schritt a) enthält.

4. Verfahren nach einem der Ansprüche Anspruch 1 bis 3, wobei die vernetzte Cellulosehydrat-Membran vor dem Beladen gemäß Schritt d) in einem weiteren Schritt b) mittels Lauge sanitisiert wird.

5. Verfahren nach einem der Ansprüche 1 bis 4, wobei die vernetzte Cellulosehydrat-Membran vor dem Beladen gemäß Schritt d) oder nach dem Sanitisieren gemäß Schritt b) in einem weiteren Schritt c) äquilibriert wird, in dem sie mit einer pufferhaltigen Lösung B, welche Polyalkylenglykol mit zwei oder drei Kohlenstoffatomen in der Wiederholungseinheit enthält, gespült wird, bis eine konstante Leitfähigkeit und/oder ein konstantes UV-Signal im Permeat gemessen wird.

6. Verfahren nach einem der Ansprüche 1 bis 5, wobei die Lösungen A und B, welche die aufzureinigenden Viren oder virusähnlichen Partikel und Verunreinigungen sowie Polyalkylenglykol mit zwei oder drei Kohlenstoffatomen in der Wiederholungseinheit enthalten, mit Hilfe eines Mischers vermischt werden.

7. Verfahren nach einem der Ansprüche 1 bis 6, wobei die Kontaktzeit der Viren oder der virusähnlichen Partikel mit der Lösung B, die Polyalkylenglykol mit zwei oder drei Kohlenstoffatomen in der Wiederholungseinheit enthält, vor dem Beladungsschritt weniger als eine Minute beträgt.

8. Verfahren nach einem der Ansprüche 1 bis 7, wobei die Konzentration an Polyalkylenglykol mit zwei oder drei Kohlenstoffatomen in der Wiederholungseinheit in der Mischung M, die mit der vernetzten Cellulosehydrat-Membran in Kontakt gebracht wird, 1 bis 20 Gew.-% bezogen auf die Gesamtmasse der Mischung M beträgt.

9. Verfahren nach einem der Ansprüche 1 bis 8, wobei das Polyalkylenglykol mit zwei oder drei Kohlenstoffatomen in der Wiederholungseinheit ausgewählt ist aus der Gruppe, bestehend aus Polyethylenglykol und Polypropylenglykol oder Gemischen daraus.

10. Verfahren nach einem der Ansprüche 1 bis 9, wobei Polyethylenglykol (PEG) mit einer mittleren Molmasse von 600 bis 10.000 Dalton ausgewählt wird.

11. Kit zur Aufreinigung von Viren oder virusähnlichen Partikeln aus einer Lösung A, mindestens umfassend eine Lösung B, die Polyalkylenglykol mit zwei oder drei Kohlenstoffatomen in der Wiederholungseinheit enthält, und eine vernetzte Cellulosehydrat-Membran.

12. Verwendung des Kits zur Aufreinigung von Viren oder virusähnlichen Partikeln nach Anspruch 11 in der Impfstoffherstellung in Form eines Einwegproduktes.

## Claims

1. A method for purifying viruses or virus-like particles from a solution, comprising the following steps:
a) providing a mixture M composed of a solution A, which contains the viruses or virus-like particles to be purified and impurities, and a solution B, which contains a polyalkylene glycol having two or three carbon atoms in the repeating unit,
d) loading a crosslinked cellulose hydrate membrane with the mixture M from step a), with the viruses or the virus-like particles attaching to the outer and inner membrane surfaces and being retained,
and
f) eluting the viruses or virus-like particles retained in the crosslinked cellulose hydrate membrane using a solution which contains no polyalkylene glycol having two or three carbon atoms in the repeating unit or which contains at least 50% less polyalkylene glycol having two or three carbon atoms in the repeating unit than the mixture M from step a), with the result that the viruses or the virus-like particles are obtained in purified form in the eluate.

2. The method according to claim 1, wherein an unpurified, clarified bioprocess solution is used as solution A.

3. The method according to claim 1 or 2, wherein step f) is preceded by a further step e), in which the loaded crosslinked cellulose hydrate membrane is washed with a solution which contains as much polyalkylene glycol having two or three carbon atoms in the repeating unit as the mixture M from step a).

4. The method according to any one of claims 1 to 3, wherein the crosslinked cellulose hydrate membrane is sanitized before the loading according to step d) in a further step b) by means of alkaline solution.

5. The method according to any one of claims 1 to 4, wherein the crosslinked cellulose hydrate membrane is equilibrated before the loading according to step d) or after the sanitization according to step b) in a further step c), in which it is flushed with a buffer-containing solution B, which contains polyalkylene glycol having two or three carbon atoms in the repeating unit, until a constant conductivity and/or a constant UV signal is measured in the permeate.

6. The method according to any one of claims 1 to 5, wherein the solutions A and B, which contain the viruses or virus-like particles to be purified and impurities as well as polyalkylene glycol having two or three carbon atoms in the repeating unit, are mixed with the aid of a mixer.

7. The method according to any one of claims 1 to 6, wherein the contact time of the viruses or the virus-like particles with the solution B, which contains polyalkylene glycol having two or three carbon atoms in the repeating unit, is less than one minute before the loading step.

8. The method according to any one of claims 1 to 7, wherein the concentration of polyalkylene glycol having two or three carbon atoms in the repeating unit in the mixture M which is contacted with the crosslinked cellulose hydrate membrane is from 1 to 20% by weight, based on the total mass of the mixture M.

9. The method according to any one of claims 1 to 8, wherein the polyalkylene glycol having two or three carbon atoms in the repeating unit is selected from the group consisting of polyethylene glycol and polypropylene glycol or mixtures thereof.

10. The method according to any one of claims 1 to 9, wherein polyethylene glycol (PEG) having an average molar mass of from 600 to 10 000 daltons is selected.

11. A kit for purifying viruses or virus-like particles from a solution A, at least comprising a solution B, which contains polyalkylene glycol having two or three carbon atoms in the repeating unit, and a crosslinked cellulose hydrate membrane.

12. The use of the kit for purifying viruses or virus-like particles according to claim 11 in vaccine production in the form of a disposable product.

## Revendications

1. Procédé de purification de virus ou de pseudo-particules virales d'une solution, comprenant les étapes suivantes:
a) fourniture d'un mélange M d'une solution A, qui contient les virus ou les pseudo-particules virales à purifier ainsi que des impuretés, et une solution B contenant un polyalkylèneglycol avec deux ou trois atomes de carbone dans le motif récurrent,
d) chargement d'une membrane réticulée en hydrate de cellulose avec le mélange M de l'étape a), les virus ou pseudo-particules virales étant fixées et retenues sur les surfaces de membranes extérieures et intérieures,
et
f) élution des virus ou pseudo-particules virales retenues dans la membrane réticulée en hydrate de cellulose avec une solution ne contenant pas de polyalkylèneglycol avec deux ou trois atomes de carbone dans le motif récurrent ou contenant au moins 50% en moins de polyalkylèneglycol avec deux ou trois atomes de carbone dans le motif récurrent que le mélange M de l'étape a), de sorte que les virus ou pseudo-particules virales dans l'éluat sont obtenues sous forme nettoyée.

2. Procédé selon la revendication 1, dans laquelle on emploie comme solution A, une solution de bioprocédé clarifiée non nettoyée.

3. Procédé selon la revendication 1 ou 2, dans laquelle une autre étape e) précède l'étape f), dans lequel la membrane réticulée en hydrate de cellulose chargée est lavée avec une solution contenant autant de polyalkylèneglycol avec deux ou trois atomes de carbone dans le motif récurrent que le mélange M de l'étape a).

4. Procédé selon l'une des revendications 1 à 3, dans lequel la membrane réticulée en hydrate de cellulose est désinfectée au moyen d'une lessive dans une autre étape b) avant le chargement conformément à l'étape d).

5. Procédé selon l'une des revendications 1 à 4, dans lequel la membrane réticulée en hydrate de cellulose est équilibrée dans une autre étape c) avant le chargement conformément à l'étape d) ou après la désinfection conformément à l'étape b), dans lequel elle est rincée avec une solution B contenant un tampon, laquelle contient du polyalkylèneglycol avec deux ou trois atomes de carbone dans le motif récurrent, jusqu'à ce qu'on mesure une conductivité constante et/ou un signal UV constant dans le perméat.

6. Procédé selon l'une des revendications 1 à 5, dans lequel les solutions A et B, contenant les virus ou pseudo-particules virales à purifier ainsi que des impuretés, ainsi que le polyalkylèneglycol avec deux ou trois atomes de carbone dans le motif récurrent, sont mélangées à l'aide d'un mélangeur.

7. Procédé selon l'une des revendications 1 à 6, dans lequel la durée de contact des virus ou des pseudo-particules virales avec la solution B, contenant le polyalkylèneglycol avec deux ou trois atomes de carbone dans le motif récurrent, est, avant l'étape de chargement, de moins d'une minute.

8. Procédé selon l'une des revendications 1 à 7, dans lequel la concentration en polyalkylèneglycol avec deux ou trois atomes de carbone dans le motif récurrent dans le mélange M, mis en contact avec la membrane réticulée en hydrate de cellulose, est de 1 à 20% en poids, sur base de la masse totale du mélange M.

9. Procédé selon l'une des revendications 1 à 8, dans lequel le polyalkylèneglycol avec deux ou trois atomes de carbone dans le motif récurrent est sélectionné parmi le groupe consistant en polyéthylèneglycol et polypropylèneglycol ou en mélanges de ceux-ci.

10. Procédé selon l'une des revendications 1 à 9, dans lequel le polyéthylèneglycol (PEG) d'une masse molaire moyenne de 600 à 10000 Dalton est sélectionné.

11. Kit de purification de virus ou de pseudo-particules virales d'une solution A, comprenant au moins une solution B, contenant le polyalkylèneglycol avec deux ou trois atomes de carbone dans le motif récurrent, et une membrane réticulée en hydrate de cellulose.

12. Utilisation du kit de purification de virus ou de pseudo-particules virales selon la revendication 11 dans la fabrication de substances à inoculer sous forme d'un produit à usage unique.
